# EUROPEAN PATENT APPLICATION

(11) **EP 0 732 124 A2**
(43) Date of publication of application: **18.09.1996**
(21) Application number: 96301738.9
(22) Date of filing: 14.03.1996
(51) Int. Cl.: A61N 1/375

(54) **Implantable electrical device having a reduced sized footprint and method of making same**

(30) Priority: 16.03.1995 US 405546
(71) Applicant: TELECTRONICS N.V., Curaçao (AN)
(72) Inventor: Yee, Arthur Kin-Wai, Pymble, N.S.W. 2073 (AU); Darkin, Don, Highlands Ranch, Colorado 80126 (US); Pham, Hai, Lakewood, Colorado 80202 (US)
(74) Representative: Waldren, Robin Michael

(57) **Abstract**

An implantable rhythm control device having a reduced sized footprint due to a header assembly that is contained within the major case profile, and a method of making same. The device includes a sealed case having two sections. The first section has an indented portion and a non-indented portion. The second section defines the footprint or major profile of the case, and the electronic components are housed within the case. The first section contains an aperture in the indented portion. A feedthrough is electrically coupled to the electronic circuitry and extends through the aperture in the case. A header assembly is located within the indented portion of the first section and is connected to the feedthrough that extends through the aperture.

## Description

### BACKGROUND OF THE INVENTION

### a. Field of Invention

This invention is directed generally to an implantable medical device having a reduced sized footprint when implanted, and in particular to the integration of an epoxy header assembly into the titanium major case profile of such an implantable medical device.

Specifically, the invention will be described herein in connection with its preferred use in an implantable rhythm control device such as a cardiac defibrillator or a cardiac pacemaker system. However, it will be apparent to those skilled in the art that the invention has broader application in other implantable electrical devices in which a housing encloses electronic circuitry which is connected to leads.

Implantable rhythm control devices, including cardiac pacemakers and defibrillators, are known in the art, and it is an on-going goal to improve the pacing and/or defibrillation efficacy of such devices as well as to decrease the size of any implanted unit. This invention relates to the integration of a header assembly into the titanium major case profile of such a medical device without protruding from the major case surface. This results in the reduction of the footprint and overall size of the implantable rhythm control device in order to better fit a sub-pectoral implant in a patient of such a device.

A small foot print for such a device is also preferred by the physician because the device-requires a smaller incision during implantation, and by the end user because it results in a more cosmetically acceptable implant.

### b. Description of the Prior Art

Conventional rhythm control devices are commonly constructed using a titanium case or two titanium half cases which, when welded together, enclose an electronic sub-assembly, a battery, and a feedthrough. The construction is typically accomplished by first connecting the electronic subassembly to the feedthrough, and fitting it into the titanium case, with the feedthrough and the case, after welding, forming a hermetic enclosure. Finally, the terminals bedded in a header are connected electrically to the feedthrough. Lastly, epoxy is used to mold or secure the header to the case.

This existing method of construction has some shortcomings. The epoxy header is at an end of the case with the feedthrough extending along a major axis. This construction results in a relatively large footprint and restricts the overall layout and interconnection of the internal electronics. The header is connected to the feedthrough and attached to the titanium case only after enclosure welding. Since the process of connecting the epoxy header to the feedthrough, if not perfect, results in an unusable product, the prior effort in enclosure welding is wasted. It would be preferable to leave the welding for the end, after it is determined that all other manufacturing steps have been successful.

Additionally, the attachment of the header to an end of the titanium case at or near the weld line restricts the design choices for the feedthrough, and makes interconnection of the epoxy header to the electrical feedthrough and welding of the case very complicated. The designer of an implantable rhythm control device is also restricted by the amount of surface area provided to lay out the feedthrough assembly.

Existing rhythm control devices are prone to another disadvantage in that the thickness of the header is limited by the thickness of the case. This in turn limits the number of cavities that can be made in one plane of the header without excessively reducing its strength. As a result, quite often the header is designed with a multiple-plane cavity configuration if several cavities are required. This gives rise to a complicated assembly process to connect the terminals and also in allowing appropriate space for set screw access. Furthermore, there is limited surface area to weld and connect the feedthrough assembly.

United States Patent No. 5,207,218 to Carpentier et al. discloses an implantable pulse generator having a generally flat case. Feedthrough wires extend through one of the major surfaces of the case, and attach to a header assembly. The header assembly is mounted on top of the major surface and extends above the major surface. The header is not protected by the case. It extends away from the case. Accordingly, the unit is configured such that the outline and contour of the unit is not smooth. The header extends outside the contour of the case. Furthermore, the design is unsuitable for ICDs which require a larger amount of circuitry and a large capacitor incorporated within the unit, such as defibrillator units. This is because the thickness of the header is added to the thickness of the case. Accordingly, the Carpentier et al. patent increases the thickness of the unit in the area of the header, rather than decreasing the thickness of the unit and does not better protect the header assembly by sheltering it within an indented portion of the case.

### OBJECTIVES AND SUMMARY OF THE INVENTION

Generally speaking, in accordance with the invention, an implantable electronic device having a reduced sized footprint is provided. The device includes a sealed case having a first section with an indented portion and a non-indented portion, and a second section. The second section defines the major case profile. An aperture in the first section of the case is located in the indented portion. At least one feedthrough member extends from the interior to the exterior of the case through this aperture. A header assembly is disposed adjacent to the indented portion of the first section. The header matches the indented portion of the case so that the header and non-indented portion of the case match the major case profile represented by the second section.

The header is thus integrated into a major surface of the case instead of being attached to an end surface of the case. Accordingly, the feedthrough extends from the major surface of the case. In addition, the electronics can be connected directly to the conductors of the feedthrough thereby eliminating extra wiring. The cavity area around the feedthrough is not restricted by the limited thickness of the case as occurs with a header assembly that is mounted on an end surface. This allows all of the cavities in the header to be laid out in a very simple configuration. In a preferred embodiment of the invention, the cavities are laid out in one single plane parallel to the case profile. The assembly and interconnections between the terminals and the feedthrough can all be performed in the same single plane.

The feedthrough can be located anywhere under the header, thus increasing the flexibility in the layout of the internal electronic assembly. The design and layout of the feedthrough is especially advantageous if it must withstand high voltage requirements, for example, for defibrillators; this can also be achieved more easily because there is a greater amount of surface area available.

The header can be fully attached to a half case and connected to the feedthrough prior to connection of the electronic assembly and enclosure welding. Furthermore, the electronics can be tested with the header assembly attached, but prior to enclosure welding. This improves manufacturability and also improves production yield.

A further advantage of the invention is that a natural alcove is provided adjacent to the header which insures a large connection region for terminating the electronic circuitry within the housing. Advantageously the alcove further provides EMI protection since the EMI-sensitive electronics can be spaced apart from the feedthrough.

Another advantage of the invention is that it requires a header which is smaller and hence requires less epoxy than existing assemblies.

It is an object of the invention to integrate the epoxy header into the major case profile without any protrusions so that the location and orientation of the implanted device is not limited by the shape and size of the header, while also providing an improved layout efficiency and a smaller footprint.

It is a further object of the invention to include in an implantable rhythm control device or other similar devices with a header which is part of the major case profile and is free from the risk of being exposed to mechanical damage.

An additional object of the invention is to provide a rhythm control device, having a smooth oval shape with an overall contour, outline and footprint of the case and header assembly which enables the internal components to blend in structurally without external irregularities and protrusions.

It is a further object of the invention to manufacture an implantable rhythm control device of smaller size. Recent trends in implantable rhythm devices are toward smaller volumes for these devices for various reasons including the limited area available in a patient for implant, ease of implant, aesthetics, and patient comfort.

Yet another object of the invention is to manufacture the rhythm control device more efficiently. The header attachment to the case, which intrinsically has low yield and is slow, can be performed before the connection of the electronic assembly and hermetic welding of the case. The present invention eliminates the scrapping or reworking common in current production practices.

It is a further object of the invention to use the large adhesion surfaces between the header and the titanium case to increase the overall adhesion strength of the epoxy header. The header blends into the indented profile of the titanium case, and is supported on two of its major surfaces, not one as in the prior art. This maximizes protection of the header from external forces and insures that after implantation, the header does not separate from the rest of the body.

Still other objects and advantages of the invention will be apparent from the specification and drawings.

The invention accordingly comprises the several steps and the relation of one or more of such steps with respect to each of the others, and the apparatus embodying features of construction, combinations of elements and arrangement of parts which are adapted to effect such steps, all as exemplified in the following detailed disclosure, and the scope of the invention will be indicated in the claims.

### BRIEF DESCRIPTION OF THE DRAWINGS

While the specification concludes with claims particularly defining and distinctly claiming the subject matter regarded as the invention herein, it is believed that the present invention will be more readily understood from the following description, taken in conjunction with the accompanying drawings, in which:
Fig. 1 is a plain view of a housing suited for a cardioverter/defibrillator assembly;
Fig. 2 is a side partial sectional view of the assembly of Figure 1;
Fig. 3 is a perspective view of the assembly of Figure 1;
Fig. 4 shows an orthogonal view of casing for a pacemaker;
Fig. 5 shows a plan view of the casing of Fig. 4;
Fig. 6 shows a side sectional view of the casing of Fig. 5 taken along line 6-6;
Fig. 7 shows a plan vie of a casing with an attached header; and
Fig. 8 shows a side sectional view of the casing of Fig. 7.

### DETAILED DESCRIPTION OF THE DRAWINGS

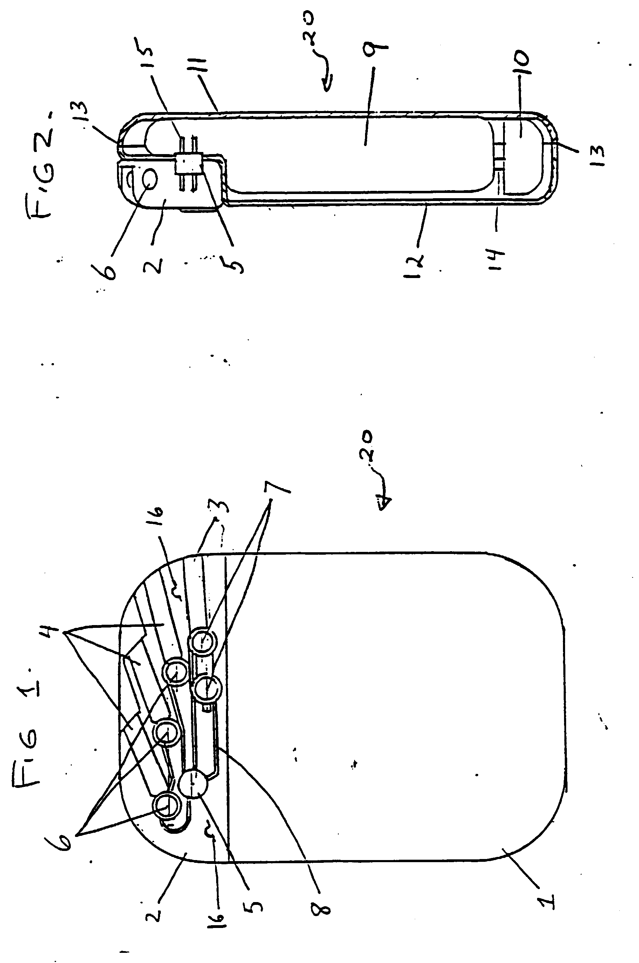

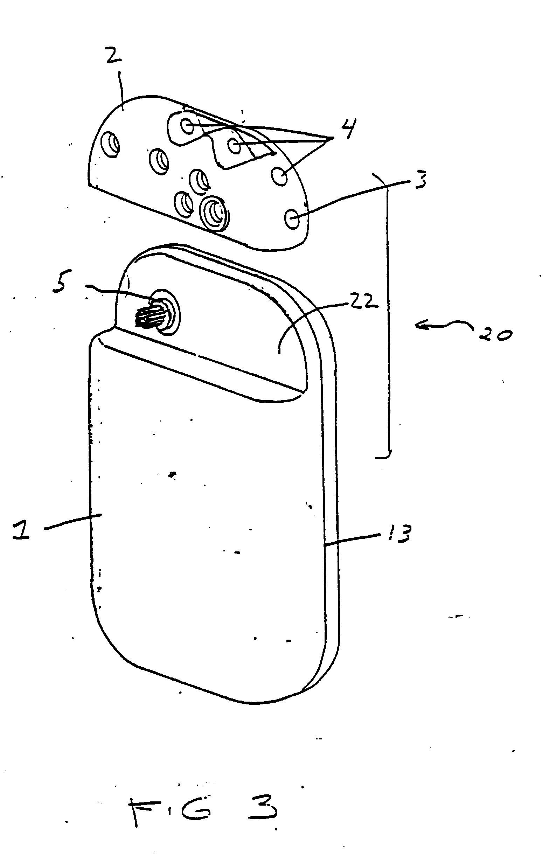

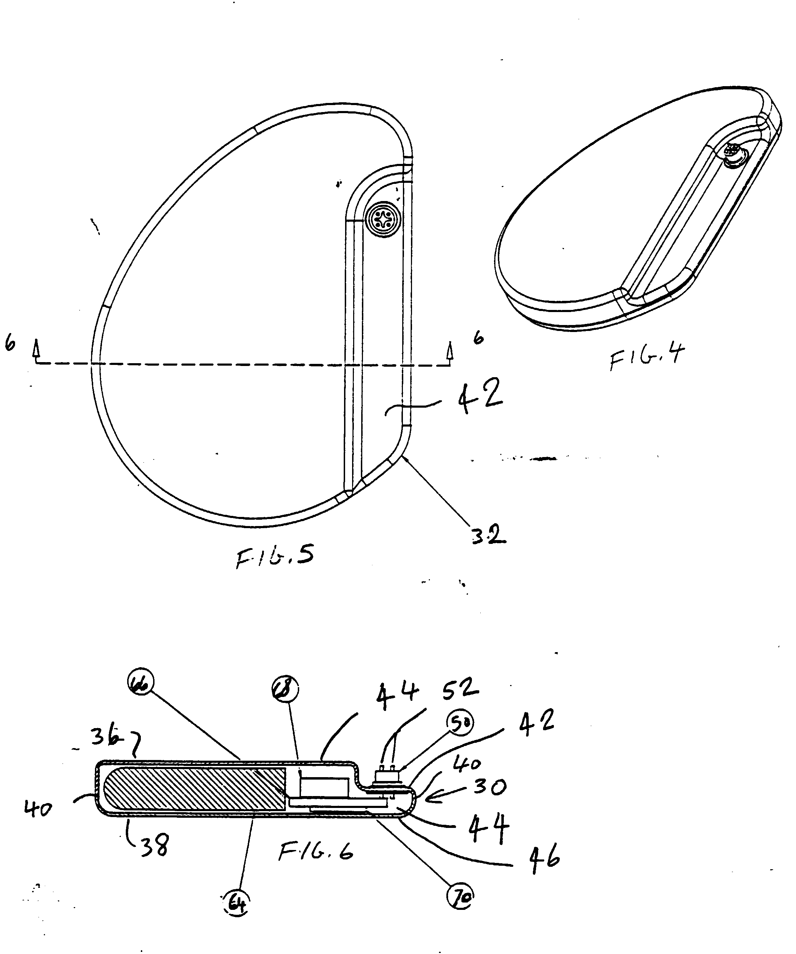

Referring to the drawings, an implantable rhythm control device such as a cardioverter/defibrillator (ICD) 20. ICD 20 includes a fully hermetic titanium case 1 made of two sections 11 and 12. Section 12 is provided with an indented portion 22 (shown in Fig. 3) to accommodate an epoxy header 2. Epoxy header 2 is shaped to match indented portion 22 of the titanium case 1. Additionally, it has a matching outside profile which blends smoothly with the portion of the titanium case 1 that is not indented. This results in a decrease of the overall footprint and thickness of the resulting ICD. The overall footprint is the case profile, defined by the contour of section 11 (the outer rectangular shape with curved corners in Fig. 1). The two sections 11 and 12 define the two major surfaces of the case.

Epoxy header 2 has at least one bipolar sense/pace lead cavity 3 and three high voltage defibrillation lead cavities 4 (although the number of lead cavities could be more or less than three depending on the specifications desired for the product). A ceramic multi-conductor feedthrough 5, for insulating the conductors from the case, is welded hermetically to titanium case 1 and interconnects the internal electronic hybrid module 9 (shown in Fig. 2), the titanium alloy defibrillation terminals 6, and the titanium alloy sense/pace terminals 7 in the epoxy header 2. The platinum terminal conductors 8 are welded to the defibrillation terminals 6, the sense/pace terminals 7, and the platinum feedthrough conductors 15. The complete epoxy header assembly is attached to titanium case 1 by adhesive and is reinforced by titanium tabs 16 (shown in Fig. 1) which are welded to case 1. Indented portion 22 on case 1 also provides added protection for epoxy header 2 from external forces.

Preferably the header is precast prior to assembly. Alternatively the header can be cast in place. The header can be made of epoxy, polyurethane, or other similar materials.

The electronic hybrid module 9 is electrically coupled to platinum conductors 15 of feedthrough 5. Electronic hybrid module 9 consists of both high voltage hybrid modules, low voltage hybrid modules, and high voltage capacitors. A battery 10 is electrically coupled to the electronic hybrid module 9 by the battery lead connections 14. Part of module 9 may be contiguous with the header behind it, as shown in Fig. 2.

Titanium case 1 preferably, as previously mentioned, includes two sections 11 and 12. Header 2 and feedthrough 5 are mounted on section 12 only. Therefore, epoxy header 2 can be mounted on section 12 of case 2 prior to the hermetic welding of section 11 to section 12. Furthermore, the unit including epoxy header 2, feedthrough 5 and the electronic circuitry can all be tested prior to sealing the case. The section 11 and section 12 are then sealed together by laser welding which is performed in a single weld plane 13 to simplify the case design and further to facilitate the welding process.

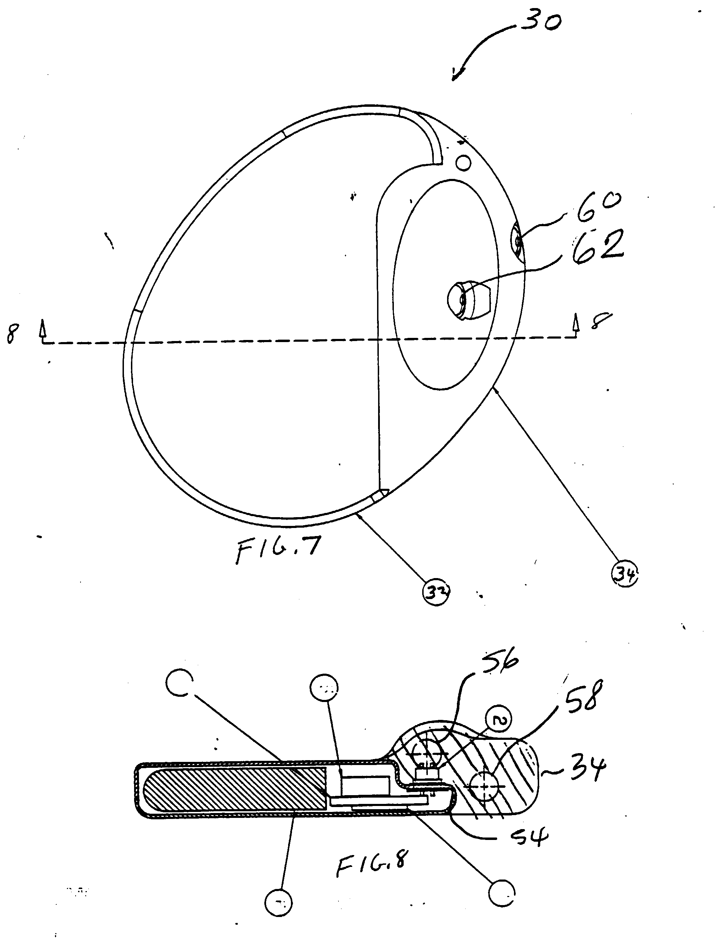

Figures 4-8 show a pacemaker 30 constructed in accordance with the present invention. The pacemaker 30 includes a titanium case 32 shown in detail in Figures 4-6 and a header shown attached to the case 32 in Figs. 7 and 8. The case 32 may be made of two sections 36 and 38 joined by a seamless weld 40 which may be accomplished, for example, by using laser welding.

Importantly, the section 36 includes a portion 42 which is generally parallel to a main wall 44 but recessed therefrom. As a result, this portion 42 forms an alcove 44 within the casing 32 with a corresponding opposing portion 48 of section 38. As seen in Figures 4-6, alcove 44 has an oblong shape and is shorter than the overall length of case 32. Alcove 46 is also about half as thick as the rest of the casing 32.

A feedthrough 50 is provided on portion 42 of casing section 36. The feedthrough 50 includes several conductors 52 for transmitting electrical signals.

Header 34 is precast or cast in place, from epoxy or other similar materials, as described above. Preferably the header 34 is formed with an indentation 54 for mating with the case 32. More specifically, the alcove 46 of case 32 is disposed in the indentation 54 of header 34.

The header 34 has a plurality of connectors 56, 58, which are electrically coupled to the conductors 52. seals 60, 62 are provided in the header to access set screws (not shown) for connectors 56, 58. Prior to the implantation of pacemaker 30, portions of the leads (not shown) of the pacing and sensing electrodes used in conjunction with the pacemaker 30 are introduced into the connectors 56, and 58 and are secured thereto through seals 60, 62 by the set screws in a well-known manner.

An important feature of the present invention is the physical structure and arrangement of the electronic components in the case 32. As shown in Figure 6, the pacemaker 30 includes a long-life battery 64 disposed in a portion of the case 32 opposite alcove 44. Adjacent to this battery 64, there is provided a substrate 66. Importantly, substrate 66 extends from the battery into alcove 44. Substrate 66 is used to mount several electronic components. These components may include a standard IC chip 68, preferably mounted on substrate 66 using wire bonds (not shown), as well as another chip 70 having contact pads (not shown) so that it can be mounted directly on the substrate without wire bonds, using 'flip-chip' technology. If necessary another board ( known as a 'daughter' board, not shown) may be provided between chip 70 and board 66. Using this technology, a thinner pacemaker is achieved.

As seen in Fig. 6, because the subtrate 66 and IC chip 70 have an overall lower height, the substrate 66 can now extend into the alcove 44, under feedthrough 50 to allow the connectors 52 to be bonded directly to the substrate 66 during assembly. Accordingly, the number of connections during assembly, and the time of assembly are reduced and the overall reliability of the system is further increased.

Although the invention has been described with reference to particular embodiments, it is to be understood that such embodiments are merely illustrative of the application of the principles of the invention. Hence numerous other modifications may be made therein and other arrangements may be devised. While there have been shown and described what are presently considered to be the preferred embodiments of this invention, it will be obvious to those skilled in the art that various other changes may be made without departing from the broader aspects of the invention.

It is also to be understood that the following claims are intended to cover all the general and specific features of the invention herein described and all statements of the scope of the invention which, as a matter of language, might be said to fall therebetween.

## Claims

1. An implantable electronic device, comprising:
a sealed case having first and second major surfaces, said first major surface having an indented portion and a non-indented portion;
electronic circuitry disposed in said sealed case;
an aperture in said indented portion;
a feedthrough member electrically coupled to said electronic circuitry and extending through said aperture to the exterior of said casing; and
a header located in said indented portion, substantially enveloping said feed-through.

2. The implantable electronic device of claim 1 wherein an exterior surface of said header lies substantially flush with the non-indented portion of said first major surface.

3. The implantable electronic device of claim 2 wherein said casing includes first and second joined sections, each of said sections defining a respective one of said first and second major surfaces.

4. The implantable electronic device of claim 3 wherein said sections are joined in a plane.

5. The implantable device of claim 1 wherein said second major portion defines a profile of said case.

6. The implantable device of claim 1 wherein said second major portion and said header cooperate to define a profile of said case.

7. The implantable electronic device of claim 1 wherein said header is attached to said case by adhesive.

8. The implantable electronic device of claim 1 wherein wire anchors couple said header to said case.

9. The implantable electronic device of claim 1 wherein said case is metallic and said header is formed of epoxy.

10. The implantable electronic device of claim 1 wherein said header includes a first surface and a second surface, said first surface of said header being shaped to substantially conform to said indented portion and said second surface of said header being substantially flush with a portion of the case connecting said indented and non-indented portions.

11. The implantable electronic device of claim 10 wherein said case includes first and second joined sections, each of said sections defining a respective one of said first and second major surfaces.

12. The implantable electronic device of claim 11 wherein said sections are joined in a plane.

13. The implantable electronic device of claim 10 wherein said header further includes a plurality of cavities, said plurality of cavities being essentially located in a single plane.

14. The implantable electronic device of claim 13 wherein each of said cavities is connected at a first end to a contact of said at least one feedthrough and at a second end to a terminal connector.

15. The implantable electronic device of claim 14 wherein said at least one feedthrough includes a ceramic outer shell and a plurality of conductors disposed therein.

16. The implantable electronic device of claim 13 wherein said case includes first and second joined sections, each of said sections defining a respective one of said first and second major surfaces.

17. The implantable electronic device of claim 16 wherein said sections are joined in a plane.

18. An implantable electronic device, comprising:
a sealed case having first and second major surfaces, said first major surface having an indented portion and a non-indented portion, and said case having electronic circuitry disposed therein;
said indented portion of said first major surface having an aperture therein;
at least one feedthrough member electrically coupled to said electronic circuitry and extending through said aperture to the exterior of said case; and
a header assembly coupled to said feedthrough and including a first surface shaped to substantially conform to said indented portion and a second surface shaped to substantially conform to a portion of the case connecting said indented and non-indented portions.

19. The implantable electronic device of claim 18 wherein the footprint of the implantable electronic device is defined by said second major surface, and said non-indented portion of said first major surface and a third surface of said header which lies substantially flush with the non-indented portion of said first major surface together conform to said footprint.

20. An implantable rhythm control device comprising: a case defined by two opposed major surfaces, at least one of said major surfaces having a surface portion being indented to form an alcove;
a substrate disposed in said case and having a substrate portion extending into said alcove;
an electronic component mounted on said substrate portion;
a feedthrough mounted on said case and including a conductor connected to said substrate; and
a header mounted on said case and surrounding said feedthrough.

21. The device of claim 20 wherein said feedthrough extends through said surface portion.

22. The device of claim 20 wherein said electronic component is mounted on said substrate without bonding wires.

23. The device of claim 20 wherein said feedthrough extends normally to said substrate.

24. A method of making an electronic device comprising the steps of:
forming a first case section and a second case section, said first case section having an aperture for receiving a feedthrough member and having an indented portion in the region of said aperture;
constructing an electronic circuit module for disposition within said case sections, said module having at least one feedthrough member;
positioning said module such that said feedthrough member extends through the aperture in said first case section;
attaching a header assembly to said indented portion so that said feedthrough member extends into said header assembly; and
sealing said first case section to said second case section to form a sealed case.

25. The method of claim 24 wherein said attaching step further includes the sub-step of electrically coupling said header assembly to said feedthrough.

26. The method of claim 24 further including the step of testing said electronic circuit after attaching said header and prior to sealing said case sections.

27. The method of claim 24 wherein said attaching step including the sub-step of adhesively bonding said header assembly to said case.

28. The method of claim 24 wherein said sealing step includes the sub-step of laser welding said sections in a single plane.

29. The method of claim 24 wherein said electronic module includes a substrate, further comprising mounting and electronic component on said module without bonding wires.
